# EUROPÄISCHE PATENTSCHRIFT

(11) **EP 3 840 801 B1**
(45) Veröffentlichungstag und Bekanntmachung des Hinweises auf die Patenterteilung: **11.11.2026**
(21) Anmeldenummer: 19735310.5
(22) Anmeldetag: 01.07.2019
(51) Int. Cl.: A61M 5/14, A61M 5/168, A61M 39/20, A61M 5/158, A61M 5/162, A61M 39/24

(54) **MEDIZINISCHES INFUSIONSSYSTEM**
MEDICAL INFUSION SYSTEM
SYSTÈME DE PERFUSION MÉDICAL

(30) Priorität: 23.08.2018 DE 102018214249
(43) Veröffentlichungstag der Anmeldung: 30.06.2021
(73) Patentinhaber: B. Braun Melsungen AG, 34212 Melsungen (DE)
(72) Erfinder: KATERKAMP, Andreas, 34212 Melsungen (DE)
(74) Vertreter: Patentanwälte Ruff, Wilhelm, Beier, Dauster & Partner mbB
(86) Internationale Anmeldenummer: PCT/EP2019/067624
(87) Internationale Veröffentlichungsnummer: WO 2020/038634

(56) Entgegenhaltungen:
- EP-A1- 3 812 000
- US-A1- 2005 171 491
- US-A1- 2010 042 048
- US-A1- 2012 145 729
- US-A1- 2014 188 089
- US-A1- 2016 030 293

## Beschreibung

Die Erfindung betrifft ein medizinisches Infusionssystem mit einer Schlauchleitung mit einem distalen Endbereich zur Verbindung mit einem Infusionsbehälter, sowie mit einem proximalen Endbereich, der einen Patientenanschluss aufweist.

Aus der US 5,954,485 A ist eine "free-flow protection"-Vorrichtung für eine Infusionspumpe eines medizinischen Infusionssystems bekannt, wobei die "free-flow protection"-Vorrichtung einen freien Durchfluss von Infusionsflüssigkeit zu einem Patientenanschluss verhindert. Die Vorrichtung ist Teil eines medizinischen Infusionssystems, bei dem Infusionsflüssigkeit aus einem Infusionsbehälter über eine Schlauchleitung zu einem Patientenanschluss fließt. Der Patientenanschluss wird im Betrieb des Infusionssystems mit einem patientenseitigen Katheter verbunden. Wenn im Betrieb des Infusionssystems der Infusionsbehälter oberhalb des Patientenanschlusses aufgehängt ist und der Patientenanschluss mit dem Katheter des Patienten verbunden ist, fließt ohne zusätzliche Maßnahmen Infusionsflüssigkeit so lange frei in eine Vene des Patienten hinein, bis sich ein Blutdruck des Patienten und der hydrostatische Druck des Infusionssystems einander angeglichen haben. Ein derartiger freier Durchfluss kann nachteilig sein, da es zu einer Übermedikation des Patienten kommen kann. Zudem können Luftblasen in der Schlauchleitung, die in den Blutkreislauf des Patienten gelangen, zu nachteiligen Folgen führen. Die US 5,954,485 offenbart eine Vorrichtung, die einen derartigen freien Fluss von Infusionsflüssigkeit verhindert. Die Vorrichtung umfasst einen Klammerbereich, der abhängig von der Stellung eines Hebels die Schlauchleitung verschließt oder freigibt. Die Vorrichtung ist einer Infusionspumpe zugeordnet, die den Durchfluss der Infusionsflüssigkeit durch die Schlauchleitung steuert.

Weiterer relevanter Stand der Technik ist aus den Dokumenten US2014/188089A1, US2012/145729A1, US2010/042048A1, US2016/030293A1, US2005/171491A1 und EP3812000A1 bekannt.

Aufgabe der Erfindung ist es, ein medizinisches Infusionssystem der eingangs genannten Art zu schaffen, das eine sichere und kontaminationsfreie Erstbefüllung des Infusionssystems ermöglicht.

Diese Aufgabe wird durch ein medizinisches Infusionssystem gemäß Anspruch 1 erfüllt. Die erfindungsgemäße Lösung ermöglicht eine Erstbefüllung der Schlauchleitung und eine Verbindung des Patientenanschlusses mit einem Patientenzugang, insbesondere einem patientenseitigen Katheter, ohne dass eine Kontamination des Anwenders oder des Patienten im Bereich des Patientenanschlusses entstehen kann. Die Luftdurchlässigkeit der Verschlusseinrichtung gewährleistet, dass bei der Erstbefüllung entstehende Luftblasen in der Schlauchleitung im Bereich des Patientenanschlusses entweichen können. Die flüssigkeitsdichte Gestaltung gewährleistet gleichzeitig, dass keine Infusionsflüssigkeit im Bereich des Patientenanschlusses bei der Erstbefüllung austreten kann. Die Verschlusseinrichtung ist vorzugsweise derart gestaltet, dass diese nur wirksam ist, bis das Infusionssystem betriebsfertig patientenseitig angeschlossen ist, um die nach dem betriebsfertigen Anschließen gewünschte Infusion von Infusionsflüssigkeit zu dem Patientenzugang, insbesondere über einen Katheter in eine Vene des Patienten, nicht zu behindern. Die Begriffe "distaler Endbereich" und "proximaler Endbereich" sind bezogen auf einen Patienten, d.h. der Patientenanschluss des Infusionssystems ist patientennah, also proximal, und der Endbereich zur Verbindung mit dem Infusionsbehälter ist patientenfern und damit distal Schlauchleitung.

Erfindungsgemäß weist die Verschlusseinrichtung eine Verschlusskappe auf, die druckdicht auf dem Patientenanschluss gehalten ist. Dadurch ist gewährleistet, dass die Verschlusskappe nicht durch einen durch eine Infusionspumpe erzeugten Förderdruck unbeabsichtigt von dem Patientenanschluss gelöst wird.

Erfindungsgemäß weist der Patientenanschluss eine Verschlussmembran auf, die den Patientenanschluss luftdurchlässig und Infusionslösung abweisend verschließt. Vorzugsweise verbleibt die Verschlusskappe mit der Verschlussmembran während einer Verbindung des Patientenanschlusses mit dem Patientenzugang an dem Patientenanschluss. Alternativ ist die Verschlussmembran derart mit dem Patientenanschluss verbunden, dass die Verschlussmembran auch nach dem Entfernen der Verschlusskappe im Bereich einer entsprechenden Durchtrittsöffnung des Patientenanschlusses verbleibt. Die Verschlussmembran wird vorteilhaft automatisch durchstoßen, sobald der Patientenanschluss mit dem Patientenzugang verbunden ist.

In weiterer Ausgestaltung der Erfindung weist der distale Endbereich eine Tropfkammer mit einem Einstechdorn auf. Der Einstechdorn ist dazu vorgesehen, die Tropfkammer mit einem Verschlussstopfen eines Infusionsbehälters zu verbinden.

In weiterer Ausgestaltung der Erfindung ist der Schlauchleitung eine Durchflusssteuereinrichtung, insbesondere in Form einer Rollenklemme, zugeordnet. Die Schlauchleitung, die Tropfkammer, die Rollenklemme und der Patientenanschluss bilden ein Überleitungssystem für das medizinische Infusionssystem, um Infusionsflüssigkeit aus dem Infusionsbehälter zu einem Patientenzugang zu leiten.

Erfindungsgemäß ist der Schlauchleitung ein Rückschlagventil mit einem Öffnungsdruck größer als ein hydrostatischer Druck des Infusionssystems zugeordnet, der in montiertem Betriebszustand in dem proximalen Endbereich der Schlauchleitung anliegt. Der hydrostatische Druck des Infusionssystems entsteht dadurch, dass der mit Infusionsflüssigkeit befüllte Infusionsbehälter in einer Höhe positioniert ist, die oberhalb eines Patientenzugangs vorgesehen ist, vorzugsweise etwa 1 m oberhalb des Patientenzugangs. Das Rückschlagventil dient als "free-flow protection", da es die Schlauchleitung verschlossen hält, solange lediglich der hydrostatische Druck des Infusionssystems innerhalb der Schlauchleitung wirksam ist. Falls der Infusionsbehälter etwa 1 m oberhalb des Patientenzugangs positioniert ist, beträgt der hydrostatische Druck innerhalb der Schlauchleitung etwa 100 mbar. Ein Öffnungsdruck des Rückschlagventils weist demzufolge vorteilhaft etwa 150 mbar auf.

Erfindungsgemäß umfasst das Infussionssystem ein Infusionspumpe, an die die Schlauchleitung angeschlossen ist und die im Betrieb auf eine zu fördernde Infusionsflüssigkeit einen Druck ausübt, der größer ist als der Öffnungsdruck des Rückschlagventils. Vorzugsweise bewirkt die Infusionspumpe im Betrieb auf die zu fördernde Infusionsflüssigkeit einen Förderdruck von 200 mbar, so dass der Betrieb der Infusionspumpe das Rückschlagventil öffnet und den Durchfluss der Infusionsflüssigkeit durch die Schlauchleitung ermöglicht. Das Rückschlagventil ist vorteilhaft der Schlauchleitung im Bereich zwischen der Infusionspumpe und dem Patientenanschluss zugeordnet.

Erfindungsgemäß öffnet die Verschlussmembran bei einem Wasserdurchbruchsdruck, der größer ist als ein Förderdruck der Infusionspumpe. Dadurch ist gewährleistet, dass bei einer Erstbefüllung der Schlauchleitung der Patientenanschluss auch dann verschlossen bleibt, wenn die Infusionspumpe ihren Betrieb aufgenommen hat. Dadurch ist eine luftblasenfreie und vollständige Erstbefüllung der Schlauchleitung ermöglicht, so dass bei einer Verbindung des Patientenanschlusses mit dem Patientenzugang eine Zufuhr von Infusionsflüssigkeit in eine Vene des Patienten erfolgen kann, ohne dass der Patientenanschluss oder der Patientenzugang kontaminiert sind. Ein Wasserdurchbruchsdruck der Verschlussmembran liegt bei einem Förderdruck der Infusionspumpe von etwa 200 mbar bei wenigstens 250 mbar.

In weiterer Ausgestaltung der Erfindung ist der Schlauchleitung ausgangsseitig der Infusionspumpe ein Drucksensor zugeordnet, der eine über den Förderdruck der Infusionspumpe hinausgehende Druckerhöhung detektiert. Der Drucksensor erfasst eine über den Förderdruck hinausgehende Druckerhöhung, die dann entsteht, wenn eine Erstbefüllung der Schlauchleitung abgeschlossen ist und die Infusionspumpe weiterhin Infusionsflüssigkeit in Richtung des Patientenanschlusses fördert.

In weiterer Ausgestaltung der Erfindung ist der Drucksensor an eine Signaleinrichtung derart angeschlossen, dass die Signaleinrichtung ein Signal abgibt, sobald eine über den Förderdruck hinausgehende Druckerhöhung detektiert wird. Die Signaleinrichtung gibt vorzugsweise ein optisches und/oder akustisches Signal aus. Das Signal dient dazu, einem Anwender mitzuteilen, dass der Erstbefüllungsvorgang abgeschlossen ist, und dass ein Antrieb der Infusionspumpe abgeschaltet werden kann.

In weiterer Ausgestaltung der Erfindung ist der Drucksensor an eine Steuereinrichtung der Infusionspumpe derart angeschlossen, dass bei Erfassung einer über den Förderdruck hinausgehenden Druckerhöhung ein Antrieb der Infusionspumpe abgeschaltet und/oder die Signaleinrichtung zur Erzeugung eines optischen und/oder akustischen Signals angesteuert wird. Hierdurch wird automatisiert die Infusionspumpe abgeschaltet, sobald der Erstbefüllungsvorgang abgeschlossen ist. Alternativ oder ergänzend gibt der Drucksensor bei Erfassen eines Drucks von 200 mbar ein entsprechendes Signal aus, das die Steuereinrichtung verarbeitet und/oder das für einen Anwender optisch und/oder akustisch erkennbar ist.

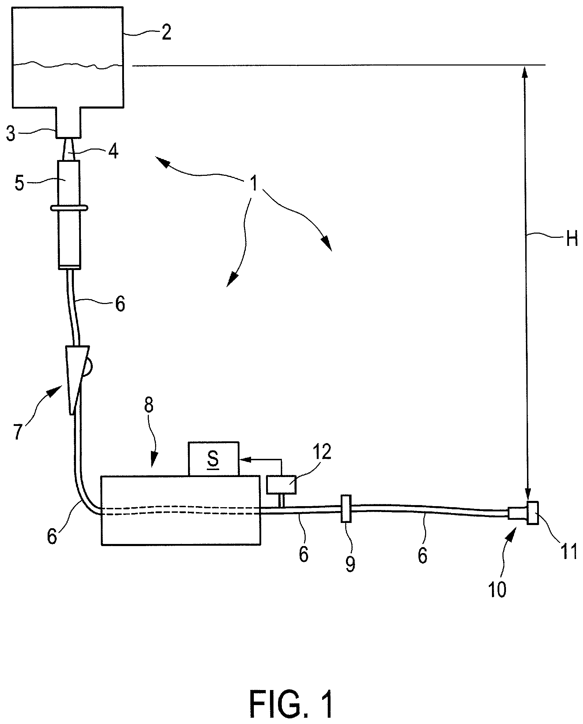

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnung dargestellt ist.

Die einzige Zeichnung zeigt schematisch eine Ausführungsform eines erfindungsgemäßen medizinischen Infusionssystems.

Ein Infusionssystem 1 weist ein Überleitungssystem zwischen einem Infusionsbehälter 2 und einem nicht dargestellten Patientenzugang auf. Das medizinische Infusionssystem 1 dient dazu, Infusionsflüssigkeit aus dem Infusionsbehälter 2 zu dem Patientenzugang zu leiten, um einem Patienten eine Medikation oder eine Ernährungslösung zu verabreichen. Als Patientenzugang ist insbesondere ein intravenöser Patientenzugang vorgesehen, mit dem das Infusionssystem 1 verbindbar ist.

Das medizinische Infusionssystem 1 weist eine flexible Schlauchleitung 6 auf, die vorteilhaft transparent ausgeführt ist. Die Schlauchleitung 6 ist an einem distalen Endbereich mit einer Tropfkammer 5 fest verbunden, die distal mit einem Einstechdorn 4 versehen ist. Der Einstechdorn 4 ist in betriebsfertig montiertem Zustand des Infusionssystems 1 kraftschlüssig in einen elastisch nachgiebigen Verschlussstopfen 3 im Bereich einer Unterseite des Infusionsbehälters 2 eingedrungen. Der Einstechdorn 4 weist wenigstens einen ins Innere des Infusionsbehälters 2 hineinragenden Durchtrittskanal auf, durch den hindurch Infusionsflüssigkeit aus dem Infusionsbehälter 2 in die Tropfkammer 5 und von dort in die Schlauchleitung 6 geleitet wird. Der Infusionsbehälter 2 ist in betriebsfertig montiertem Zustand, insbesondere in einem Gestell, stationär aufgehängt. Das Gestell ist vorteilhaft mit Rollen versehen, um den Infusionsbehälter 2 einschließlich des Infusionssystems 1 auf einem Untergrund verfahren zu können.

In Durchflussrichtung des Infusionssystems 1 schließt an die Tropfkammer 5 eine Rollenklemme 7 an, die dazu vorgesehen ist, manuell einen Durchfluss der Infusionsflüssigkeit durch die Schlauchleitung 6 zu steuern. Dem Infusionssystem 1 ist zudem ein nachfolgend näher beschriebenes Rückschlagventil 9 sowie an einem proximalen Endbereich der Schlauchleitung 6 ein Patientenanschluss 10 zugeordnet. Der Patientenanschluss 10 ist durch eine Verschlusskappe 11 verschlossen.

In betriebsfertig montiertem Zustand ist die Schlauchleitung 6 an eine Infusionspumpe 8 angeschlossen, die mit einem nicht näher dargestellten Antrieb versehen ist, um durch mechanisches Einwirken auf einen Mantel der Schlauchleitung 6 eine Förderwirkung für die durch die Schlauchleitung 6 hindurchgeleitete Infusionsflüssigkeit zu erzielen. Hierzu ist die Schlauchleitung 6 in grundsätzlich bekannter Weise in einen entsprechenden Aufnahmebereich der Infusionspumpe 8 eingelegt und in dem Aufnahmebereich fixiert.

Der Schlauchleitung 6 ist schließlich ausgangsseitig der Infusionspumpe 8 ein Drucksensor 12 zugeordnet, der an eine nachfolgend näher beschriebene Steuereinrichtung S angeschlossen ist.

Der Patientenanschluss 10 ist in nicht näher dargestellter Weise mit einer Verschlussmembran versehen, die den Patientenanschluss luftdurchlässig und Infusionslösung abweisend, d.h. flüssigkeitsdicht, verschließt. Die Verschlussmembran ist entweder direkt einer Durchtrittsöffnung des Patientenanschlusses 10 oder der Verschlusskappe 11 zugeordnet. In beiden Fällen dient die Verschlussmembran dazu, das Infusionssystem 1 und damit die Schlauchleitung 6 proximal endseitig flüssigkeitsdicht zu verschließen, gleichzeitig jedoch Luft hindurchzulassen. Falls die Verschlussmembran der Verschlusskappe 11 zugeordnet ist, verbleibt die Verschlusskappe bei einer Verbindung des Patientenanschlusses 10 mit dem Patientenzugang vorzugsweise an dem Patientenanschluss 10, um bei einer Verbindung automatisch ein Durchtrennen der Verschlussmembran zu erzielen, ohne dass Infusionsflüssigkeit an einer Verbindungsstelle zwischen Patientenanschluss 10 und Patientenzugang austritt. Vorzugsweise sind der Patientenanschluss 10 und der Patientenzugang mit zueinander komplementären Luer-Lock-Anschlüssen versehen. Falls die Verschlussmembran dem Patientenanschluss 10 direkt zugeordnet ist, kann die Verschlusskappe 11 von dem Patientenanschluss 10 entfernt werden, ohne dass Infusionsflüssigkeit aus dem Patientenanschluss 10 austreten kann. Denn die Verschlussmembran verschließt den Patientenanschluss 10 weiterhin, bis der Patientenanschluss 10 mittels einer entsprechenden Luer-Lock-Verbindung fest mit dem Patientenzugang verbunden ist. Bei der Verbindung wird die Verschlussmembran vorteilhaft durchstochen oder in anderer Weise durchtrennt, wodurch ein Durchfluss für die Infusionsflüssigkeit freigegeben wird. Die Luftdurchlässigkeit der Verschlussmembran gewährleistet, dass bei einer Erstbefüllung des Infusionssystems 1 Luftblasen durch die Schlauchleitung 6 zum Patientenanschluss 10 hindurchgeleitet und am Patientenanschluss 10 entweichen können, so dass eine luftblasenfreie Erstbefüllung mit Infusionsflüssigkeit ermöglicht ist.

Für die Inbetriebnahme des Infusionssystems 1 wird der Einstechdorn 4 in den Verschlussstopfen 3 des Infusionsbehälters 2 eingefüllt. Der Infusionsbehälter 2 ist bereits stationär aufgehängt in einer Höhe H, die vorzugsweise etwa 1 m oberhalb des Patientenzugangs vorgesehen ist. Der Einstechdorn 4 wird von unten her in den Verschlussstopfen 3 eingedrückt, bis Infusionsflüssigkeit durch den Durchtrittskanal des Einstechdorns 4 in die Tropfkammer 5 fließen kann. Die Rollenklemme 7 ist geöffnet. Die Schlauchleitung 6 ist im Bereich der mit einem elektrischen Antrieb versehenen Infusionspumpe 8 montiert.

Das Rückschlagventil 9 weist einen Öffnungsdruck auf, der größer ist als ein durch den Höhenunterschied zwischen dem aufgehängten Infusionsbehälter 2 und dem Patientenzugang, d.h. dem Patientenanschluss 10, entstehender hydrostatischer Druck. Solange daher die Infusionspumpe 8 nicht aktiviert ist, verschließt das Rückschlagventil 9 die Schlauchleitung 6 in einem Bereich zwischen der Infusionspumpe 8 und dem Patientenanschluss 10. Das Rückschlagventil 9 definiert eine "free-flow protection"-Vorrichtung. Der Öffnungsdruck des Rückschlagventils 9 ist jedoch geringer als ein Förderdruck der Infusionspumpe 8, sobald die Infusionspumpe 8 aktiviert ist. Nach Aktivierung der Infusionspumpe 8 öffnet demzufolge das Rückschlagventil 9 aufgrund des dann in der Schlauchleitung 6 erhöhten Förderdrucks für die Infusionsflüssigkeit, so dass die Infusionsflüssigkeit bis zum Patientenanschluss 10 gefördert wird. Der Patientenanschluss 10 ist durch die Verschlussmembran verschlossen. Gemeinsam mit der Infusionsflüssigkeit werden auch etwaige Luftblasen in Richtung des Patientenanschlusses 10 gefördert. Die entsprechende Luft kann aufgrund der Luftdurchlässigkeit der Verschlussmembran im Bereich des Patientenanschlusses 10 in die Umgebung austreten. Gleichzeitig ist die Verschlussmembran Infusionslösung abweisend ausgeführt, so dass Infusionslösung im Bereich der Verschlussmembran des Patientenanschlusses 10 aufgestaut wird. Bei weiterer Förderung von Infusionsflüssigkeit durch den Betrieb der Infusionspumpe 8 erhöht sich somit zwangsläufig in der Schlauchleitung 6 der Druck. Die entsprechende Druckerhöhung wird durch den Drucksensor 12 überwacht, der ausgangsseitig der Infusionspumpe 8 vorgesehen ist. Sobald sich der Druck innerhalb der Schlauchleitung über den normalen Förderdruck der Infusionspumpe 8 erhöht, gibt der Drucksensor 12 ein Signal an die Steuereinrichtung S, die dann einen nicht dargestellten akustischen und/oder optischen Signalgeber ansteuert und/oder den Antrieb der Infusionspumpe 8 abschaltet. Falls lediglich der akustische und/oder optische Signalgeber angesteuert wird, muss der Anwender, insbesondere das Klinikpersonal, abhängig vom Erfassen des entsprechenden Signals den Antrieb der Infusionspumpe 8 ausschalten. Falls die Steuereinrichtung S direkt den Antrieb der Infusionspumpe 8 ansteuert, kann bei Erfassung einer entsprechenden Druckerhöhung ein automatisches Abschalten des Antriebs der Infusionspumpe 8 erfolgen.

Falls der Höhenunterschied H 1 m beträgt, beträgt der hydrostatische Druck innerhalb des Infusionssystems 1 100 mbar. Das Rückschlagventil 9 weist einen Öffnungsdruck von etwa 150 mbar auf. Ein Förderdruck, der durch Aktivierung der Infusionspumpe 8 erzeugt wird, liegt vorzugsweise bei mehr als 150 mbar und vorteilhaft bei maximal 200 mbar. Der Drucksensor 12 ist vorteilhaft derart ausgelegt, dass er bei einem Druck von mehr als 200 mbar ein entsprechendes Signal an die Steuereinrichtung S aussendet. Ein Wasserdurchbruchsdruck der Verschlussmembran liegt bei den zuvor angegebenen Werten dann vorteilhaft bei wenigstens 250 mbar.

Damit ist eine sichere und luftblasenfreie Erstbefüllung des Infusionssystems 1 sichergestellt. Nun kann der Patientenanschluss 10 mit dem Patientenzugang verbunden werden, wodurch die Verschlussmembran öffnet und ein Durchfluss für die Infusionsflüssigkeit zu dem Patientenzugang hin freigegeben wird.

## Patentansprüche

1. Medizinisches Infusionssystem (1) mit einer Schlauchleitung (6) mit einem distalen Endbereich zur Verbindung mit einem Infusionsbehälter (2), sowie mit einem proximalen Endbereich, der einen Patientenanschluss (10) aufweist, wobei der Patientenanschluss (10) mit einer Verschlusseinrichtung versehen ist, die luftdurchlässig und flüssigkeitsdicht gestaltet ist, wobei der Schlauchleitung (6) ein Rückschlagventil (9) des Infusionssystems (1) zugeordnet ist, mit einem Öffnungsdruck größer einem hydrostatischen Druck des Infusionssystems (1), der in montiertem Betriebszustand des Infusionssystems (1) in dem proximalen Endbereich der Schlauchleitung (6) anliegt, **dadurch gekennzeichnet, dass** das Infusionssystem (1) weiterhin eine Infusionspumpe (8) aufweist, an die die Schlauchleitung (6) angeschlossen ist, wobei die Infusionspumpe (8) mit einem Antrieb versehen ist, um durch mechanisches Einwirken auf einen Mantel der Schlauchleitung (6) eine Förderwirkung für eine durch die Schlauchleitung (6) hindurchgeleitete Infusionsflüssigkeit zu erzielen, wobei die Schlauchleitung (6) in einen Aufnahmebereich der Infusionspumpe (8) eingelegt und in dem Aufnahmebereich fixiert ist, und dass das Rückschlagventil (9) in der Schlauchleitung (6) in einem Bereich zwischen der Infusionspumpe (8) und dem Patientenanschluss (10) positioniert ist.

2. Medizinisches Infusionssystem nach Anspruch 1, **dadurch gekennzeichnet, dass** die Infusionspumpe (8) im Betrieb auf eine zu fördernde Infusionsflüssigkeit einen Druck ausübt, der größer ist als der Öffnungsdruck des Rückschlagventils (9).

3. Medizinisches Infusionssystem nach Anspruch 1 oder 2, wobei die Verschlusseinrichtung eine Verschlusskappe (11) aufweist, die druckdicht auf dem Patientenanschluss (10) gehalten ist, wobei der Patientenanschluss (10) oder die Verschlusskappe (11) eine Verschlussmembran aufweisen, die den Patientenanschluss (10) luftdurchlässig und Infusionslösung abweisend verschließt, **dadurch gekennzeichnet, dass** die Verschlussmembran bei einem Wasserdurchbruchsdruck öffnet, der größer ist als ein Förderdruck der Infusionspumpe (8).

4. Medizinisches Infusionssystem nach einem der Ansprüche 2 oder 3, **dadurch gekennzeichnet, dass** an die Schlauchleitung (6) ausgangsseitig der Infusionspumpe (8) ein Drucksensor (12) angeschlossen ist, der eine über den Förderdruck der Infusionspumpe (8) hinausgehende Druckerhöhung detektiert.

5. Medizinisches Infusionssystem nach Anspruch 4, **dadurch gekennzeichnet, dass** der Drucksensor (12) an eine Signaleinrichtung derart angeschlossen ist, dass die Signaleinrichtung ein Signal abgibt, sobald eine über den Förderdruck hinausgehende Druckerhöhung detektiert wird.

6. Medizinisches Infusionssystem nach Anspruch 5, **dadurch gekennzeichnet, dass** die Signaleinrichtung dazu vorgesehen ist, ein optisches und/oder akustisches Signal auszugeben.

7. Medizinisches Infusionssystem nach einem der Ansprüche 4 bis 6, **dadurch gekennzeichnet, dass** der Drucksensor (12) an eine Steuereinrichtung (S) der Infusionspumpe (8) derart angeschlossen ist, dass bei Erfassung einer über den Förderdruck hinausgehenden Druckerhöhung ein Antrieb der Infusionspumpe (8) abgeschaltet und/oder die Signaleinrichtung zur Erzeugung eines optischen und/oder akustischen Signals angesteuert wird.

## Claims

1. Medical infusion system (1) having a hose line (6) with a distal end region for connection to an infusion reservoir (2) and with a proximal end region having a patient connection port (10), wherein the patient connection port (10) is provided with a closure device which is air-permeable and liquid-tight, wherein the hose line (6) is assigned a check valve (9) of the infusion system (1) that has an opening pressure greater than a hydrostatic pressure of the infusion system (1) that is present in the proximal end region of the hose line (6) in the assembled operating state of the infusion system (1), **characterized in that** the infusion system (1) additionally has an infusion pump (8) to which the hose line (6) is connected, wherein the infusion pump (8) is provided with a drive for achieving a conveying action for an infusion liquid conducted through the hose line (6) by mechanically acting on a sheath of the hose line (6), wherein the hose line (6) is inserted into a receiving region of the infusion pump (8) and fixed in the receiving region, and **in that** the check valve (9) is positioned in the hose line (6) in a region between the infusion pump (8) and the patient connection port (10).

2. Medical infusion system according to Claim 1, **characterized in that** the infusion pump (8) during operation exerts a pressure on an infusion liquid to be delivered that is greater than the opening pressure of the check valve (9).

3. Medical infusion system according to Claim 1 or 2, wherein the closure device has a closure cap (11) which is held pressure-tightly on the patient connection port (10), wherein the patient connection port (10) or the closure cap (11) has a closure membrane which closes the patient connection port (10) in such a way that the latter is permeable to air and repellent to infusion solution, **characterized in that** the closure membrane opens at a water breakthrough pressure greater than a delivery pressure of the infusion pump (8).

4. Medical infusion system according to either of Claims 2 and 3, **characterized in that** the hose line (6) on the output side of the infusion pump (8) has connected to it a pressure sensor (12) which detects an increase in pressure that exceeds the delivery pressure of the infusion pump (8).

5. Medical infusion system according to Claim 4, **characterized in that** the pressure sensor (12) is connected to a signal device in such a way that the signal device emits a signal as soon as an increase in pressure that exceeds the delivery pressure is detected.

6. Medical infusion system according to Claim 5, **characterized in that** the signal device is intended to output an optical and/or acoustic signal.

7. Medical infusion system according to any of Claims 4 to 6, **characterized in that** the pressure sensor (12) is connected to a control device (S) of the infusion pump (8) in such a way that, when an increase in pressure that exceeds the delivery pressure is detected, a drive of the infusion pump (8) is switched off and/or the signal device is activated to generate an optical and/or acoustic signal.

## Revendications

1. Système de perfusion médical (1) comprenant une tubulure (6) présentant une zone d'extrémité distale destinée à être reliée à un récipient de perfusion (2), ainsi qu'une zone d'extrémité proximale qui présente un raccord patient (10), le raccord patient (10) étant pourvu d'un dispositif de fermeture qui est perméable à l'air et étanche aux liquides, un clapet anti-retour (9) du système de perfusion (1) étant associé à la tubulure (6),
présentant une pression d'ouverture supérieure à une pression hydrostatique du système de perfusion (1), qui règne, à l'état de fonctionnement monté du système de perfusion (1), dans la zone d'extrémité proximale de la tubulure (6), **caractérisé en ce que** le système de perfusion (1) présente en outre une pompe à perfusion (8) à laquelle la tubulure (6) est raccordée, la pompe à perfusion (8) étant pourvue d'un entraînement destiné à produire, par action mécanique sur une paroi de la tubulure (6), un effet de pompage pour un liquide de perfusion acheminé à travers la tubulure (6), la tubulure (6) étant insérée dans une zone de réception de la pompe à perfusion (8) et fixée dans la zone de réception, et **en ce que** le clapet anti-retour (9) est positionné dans la tubulure (6), dans une zone située entre la pompe à perfusion (8) et le raccord patient (10).

2. Système de perfusion médical selon la revendication 1, **caractérisé en ce que** la pompe à perfusion (8) exerce, pendant le fonctionnement, sur un liquide de perfusion à pomper une pression supérieure à la pression d'ouverture du clapet anti-retour (9).

3. Système de perfusion médical selon la revendication 1 ou 2, dans lequel le dispositif de fermeture présente un capuchon de fermeture (11) qui est maintenu de manière étanche à la pression sur le raccord patient (10), le raccord patient (10) ou le capuchon de fermeture (11) présentant une membrane de fermeture qui ferme le raccord patient (10) de manière à être perméable à l'air et à repousser la solution de perfusion, **caractérisé en ce que** la membrane de fermeture s'ouvre à une pression de percée de l'eau supérieure à une pression de pompage de la pompe à perfusion (8).

4. Système de perfusion médical selon l'une des revendications 2 ou 3, **caractérisé en ce qu'**un capteur de pression (12) est raccordé à la tubulure (6) du côté de la sortie de la pompe à perfusion (8), lequel capteur détecte une augmentation de pression dépassant la pression de pompage de la pompe à perfusion (8).

5. Système de perfusion médical selon la revendication 4, **caractérisé en ce que** le capteur de pression (12) est raccordé à un dispositif de signalisation de telle sorte que le dispositif de signalisation émet un signal dès qu'une augmentation de pression dépassant la pression de pompage est détectée.

6. Système de perfusion médical selon la revendication 5, **caractérisé en ce que** le dispositif de signalisation est prévu pour émettre un signal optique et/ou acoustique.

7. Système de perfusion médical selon l'une des revendications 4 à 6, **caractérisé en ce que** le capteur de pression (12) est raccordé à un dispositif de commande (S) de la pompe à perfusion (8) de telle sorte que, lors de la détection d'une augmentation de pression dépassant la pression de pompage, un entraînement de la pompe à perfusion (8) est arrêté et/ou le dispositif de signalisation est commandé afin de générer un signal optique et/ou acoustique.
